# EUROPÄISCHE PATENTSCHRIFT

(11) **EP 1 005 293 B1**
(45) Veröffentlichungstag und Bekanntmachung des Hinweises auf die Patenterteilung: **05.09.2001**
(21) Anmeldenummer: 99931083.2
(22) Anmeldetag: 17.06.1999
(51) Int. Cl.: A61B 17/00, A61B 1/005

(54) **MEDIZINISCHES INSTRUMENT ZUR ENDOSKOPISCHEN ENTNAHME DER VENA SAPHENA MAGNA**
MEDICAL INSTRUMENT FOR ENDOSCOPIC REMOVAL OF THE VENA SAPHENA MAGNA
INSTRUMENT MEDICAL POUR RETIRER PAR VOIE ENDOSCOPIQUE LA VEINE SAPHENE INTERNE

(30) Priorität: 19.06.1998 DE 19827360
(43) Veröffentlichungstag der Anmeldung: 07.06.2000
(73) Patentinhaber: Karl Storz GmbH & Co. KG, 78532 Tuttlingen (DE)
(72) Erfinder: SCHÖLLHORN, Joachim, D-79104 Freiburg (DE); BEYERSDORF, Friedhelm, D-79199 Kirchzarten (DE); LUTZ, Christoph, D-79104 Freiburg (DE)
(74) Vertreter: Heuckeroth, Volker
(86) Internationale Anmeldenummer: EP9904185
(87) Internationale Veröffentlichungsnummer: WO9966842

(56) Entgegenhaltungen:
- US-A- 5 373 840
- US-A- 5 643 221
- US-A- 5 667 480
- "Instrumente zur endoskopischen Entnahme der Vena Saphena Magna" ENDO WORLD, Nr. CHIR 4/1-D, 1998, XP002118495 Tuttlingen, DE in der Anmeldung erwähnt

## Beschreibung

Die Erfindung betrifft ein medizinisches Instrument zur endoskopischen Entnahme der Vena Saphena Magna, mit einem langerstreckten Schaft, der am distalen Ende eine Spatelspitze aufweist, und in dessen proximalem Bereich ein seitlich abstehender Handgriff angeordnet ist, und ferner mit einer Endoskopoptik, die eine Okularmuschel aufweist, die am proximalen Ende des Instrumentes angeordnet ist, wobei sich von einer Längsmittelachse des Schafts eine gedachte erste Halbebene erstreckt, die eine Längsmittelachse der Okularmuschel einschließt.

Ein Instrument der eingangs genannten Art ist aus der von der Karl Storz GmbH & Co., Tuttlingen, herausgegebenen DE-Firmenschrift "Endo World", CHIR Nr. 4-D, 1997, bekannt. Auf Seite 3 dieser Firmenschrift ist ein derartiges Instrument unter der Bezeichnung "Optischer Retraktor" abgebildet.

Die Vena Saphena Magna ist eine große Beinvene, die an der medialen, d.h. der inneren Seite des Beines von dem inneren Knöchel entlang des Unter- und des Oberschenkels bis zur Leistengegend verläuft.

Die Vena Saphena Magna wird häufig entnommen, um sie als Transplantat in der Koronar- und Gefäßchirurgie einzusetzen. Bei herkömmlichen Operationsmethoden zur Entnahme der Vena Saphena Magna wird entweder ein einziger langer Einschnitt entlang der Innenseite des Beines angebracht, oder es werden mehrere kürzere, von einander getrennte Einschnitte gesetzt. Mittels durch diese Einschnitte oder Inzisionen eingeführte Instrumente, sogenannte Venen-Dissektoren, wird die Vena Saphena Magna von dem umliegenden Bindegewebe und ihren seitlichen Gefäßabzweigungen befreit. Die freigelegte und isolierte Vene wird dann durch den Einschnitt bzw. die Einschnitte entnommen. Diese bislang übliche Art der Entnahme über eine einzige lange bzw. mehrere separate Inzisionen birgt jedoch die Gefahr der Verletzung des medialen Lymphbündels und damit der Infektion des Operationsgebietes in sich.

In dem Artikel "Minimal-invasive, video-assisted vein harvesting for cardiac and vascular surgical procedures" von Lutz et.al. (1997), in European Journal of Cardio-Thoracic Surgery 12, Seiten 519-521, wird ein alternatives Verfahren zur Entnahme der Vena Saphena Magna beschrieben, bei dem die Vene unter endoskopischer Kontrolle auf minimal-invasivem Wege entnommen wird. Hierzu wird lediglich ein einziger kleiner, 2-3 cm langer Einschnitt in der Nähe des Kniegelenks eingebracht. Durch diesen Einschnitt wird das eingangs genannte Instrument nach oben entlang des Oberschenkelabschnitts der Vene bis in die Leistengegend und nach unten entlang des Unterschenkelabschnitts der Vene bis zum inneren Fußknöchel eingeführt. Dabei wird die Vene von Bindegewebe und seitlichen Gefäßabzweigungen befreit und die gesamte Vene durch den einzigen Einschnitt im Kniebereich herausgezogen. Diese endoskopische Entnahmetechnik ist im Vergleich zu der zuvor beschriebenen früheren Entnahmeart wegen des nur einen erforderlichen Einschnittes gewebeschonend, und die postoperativen Beschwerden des Patienten und die Gefahr eines Wundinfektes sind erheblich geringer. Außerdem erfolgt die Entnahme nach diesem neueren Verfahren stets unter endoskopischer Sichtkontrolle.

Das aus der eingangs genannten DE-Firmenschrift "Endo World" bekannte Instrument, das für den zuvor beschriebenen Eingriff geeignet ist, weist einen langerstreckten Schaft auf, der an seinem proximalen Ende einen seitlich abstehenden Handgriff sowie eine zu einer Endoskopoptik gehörende Okularmuschel trägt. Der Schaft ist vom proximalen bis zum distalen Ende, an dem eine schmale, in distaler Richtung verjüngte und leicht gewölbte Spatelspitze ausgebildet ist, als in etwa nierenförmige Rinne zur äußeren Aufnahme eines Optikschaftes der Endoskopoptik ausgebildet, d.h. der Endoskopschaft liegt in der Rinne außen am Schaft an. Die Endoskopoptik aus Optikschaft und Okular mit Okularmuschel ist von dem Schaft abnehmbar, in dem die Endoskopoptik nach proximal durch einen Befestigungsabschnitt des Handgriffs hindurch vom Schaft abgezogen wird. Der Schaft des medizinischen Instruments ist etwa 30 cm lang, um die Venenenden von dem einzigen Einschnitt im Kniebereich aus erreichen zu können.

Bei dem bekannten Instrument ist der Handgriff an dem Schaft so befestigt, daß der Schaft im Bereich des Handgriffs verbreitert ist, d.h. daß die vom Handgriff abgewandte Außenseite des Instruments im Bereich des Ansatzes des Handgriffs eine Stufe aufweist. Weiterhin ist die Okularmuschel am proximalen Ende des Schaftes so angeordnet, daß die Längsmittelachse der Okularmuschel in geradliniger coaxialer Verlängerung der Längsmittelachse des Schaftes des Instrumentes verläuft, so daß die Okularmuschel umfänglich den Schaft allseitig überragt.

Diese Bauart des bekannten Instruments ist jedoch bei einem operativen Eingriff zur Entnahme der Vena Saphena Magna nachteilig.

Bei dem endoskopischen Eingriff zur Entnahme der Vena Saphena Magna wird das Instrument nämlich durch den Einschnitt im Kniebereich eingeführt und entlang der Vene nach oben zur Leistengegend bzw. nach unten in den Knöchelbereich vorangeschoben.

Um die Entnahme der gesamten Vene durch einen einzigen Einschnitt zu ermöglichen, muß die ganze Länge des medizinischen Instruments ausgenutzt werden, denn das Instrument muß vom Knie aus bis in die Leistengegend bzw. bis zum Fußknöchel entlang der Vene vorgeschoben werden. Da die Vene dicht unter der Haut verläuft, muß der Schaft des Instrumentes nahezu parallel zur Hautoberfläche vorangeschoben werden, so daß der beim Voranschieben des Schaftes entlang der Vene nach außerhalb der Inzision befindliche Abschnitt des Schafts möglichst eng am Bein anliegend gehalten vorwärts geschoben werden muß.

Bei dem bekannten Instrument ist dadurch, daß der Befestigungsabschnitt des Handgriffs und das Okular wie vorstehend beschrieben im proximalen, außerhalb des Körpers verbleibenden Bereich den Schaft seitlich überragen, das Instrument in seinem proximalen Bereich über dem Schaft an der am Bein anliegenden Außenseite des Instrumentes wesentlich verbreitert. Diese Verbreiterung verhindert jedoch ein enges Anliegen des Instrumentes am Bein des Patienten mit der Folge, daß die Spatelspitze nicht dicht unter der Hautoberfläche entlang der Vene nach vorn geschoben werden kann. Dies ist zumindest dann der Fall, wenn das Instrument bereits weit in das Operationsgebiet vorangeschoben ist. Durch die Verbreiterung des Instruments im proximalen Bereich an der von dem Handgriff abgewandten Außenseite ist es somit beinahe unmöglich, die Spatelspitze tief im Operationsgebiet weiter parallel zur Hautoberfläche entlang der Vena Saphena Magna zu führen, vielmehr dringt die Spatelspitze in tieferliegendes Gewebe ein und kann dabei zu unerwünschten Verletzungen von unbeteiligtem Gewebe führen. Um diese Gefahr zu vermeiden, kann das bekannte Instrument nur bis zu einer bestimmten Einschubtiefe des Schaftes in das Operationsgebiet verwendet werden. Darüber hinaus besteht ein weiterer Nachteil des bekannten Instrumentes darin, daß mit zunehmender Einschubtiefe des Schaftes in die Inzision die zur Beobachtung der Operation durch die Endoskopoptik an das Okular angeschlossene Kamera ab einer bestimmten Einschubtiefe so dicht am Bein des Patienten anliegt, daß die Kamera, deren Gehäuse eine quer zur Schaftachse breitere Abmessung als der Schaft selbst aufweist, ein paralleles subkutantes Voranschieben des Instrumentes behindert. Außerdem behindert die Kamera das Einführen weiterer Hilfsinstrumente in die Inzision. Somit ist auch die Handhabung des bekannten Instrumentes erschwert.

Aus der US 5,667,480 ist ebenfalls ein Instrument zur endoskopischen Entnahme der Vena saphena Magna bekannt, bei dem die vorgenannten Nachteile ebenfalls bestehen, nämlich daß der Schaft im Bereich des Ansatzes des Handgriffes verbreitert ist, und daß das Okular axial ausgerichtet ist.

Die US 5,373,840 offenbart ein vergleichbares Instrument, mit einem seitlich vom Schaft abstehenden Handgriff und mit einer integrierten Endoskopoptik, die das Beobachtungsbild direkt auf einen Monitor überträgt. Anstelle des Monitors kann auch ein Okular in klassischer Weise vorgesehen sein, jedoch ist nicht angegeben, wie das Okular dann anzuordnen wäre.

Vor diesem Hintergrund ist es Aufgabe der vorliegenden Erfindung, ein medizinisches Instrument der eingangs genannten Art bereitzustellen, das es erlaubt, die Vena Saphena Magna durch einen möglichst kleinen Einschnitt am Körper des Patienten zu entnehmen, wobei die Spatelspitze des Instrumentes möglichst über die gesamte Einschubtiefe des Schaftes in die Inzision dicht unter der Hautoberfläche entlang der Vene führbar sein soll.

Diese Aufgabe wird hinsichtlich des eingangs genannten medizinischen Instrumentes dadurch gelöst, daß der Handgriff so mit dem Schaft verbunden ist, daß eine von dem Handgriff abgewandte Außenseite des Instrumentes vom distalen bis zum proximalen Ende eine von Vorsprüngen im wesentlichen freie Fläche aufweist, und daß die Okularmuschel bezüglich der Längsmittelachse des Schafts schräg gerichtet angeordnet ist, wobei sich von der Längsmittelachse des Schafts eine gedachte zweite Halbebene erstreckt, die eine Längsmittelachse des Handgriffs einschließt, und daß die gedachte erste Halbebene (siehe erster Absatz dieser Beschreibung) und die gedachte zweite Halbebene einen Winkel von weniger als 90° einschließen.

Durch die erfindungsgemäße Bauweise erhält das medizinische Instrument an seiner vom Handgriff abgewandten Außenseite eine vom proximalen Ende bis zum Beginn der distalen Spatelspitze verlaufende einheitliche Fläche, die frei von Vorsprüngen ist, die somit ein enges Anliegen des proximalen Bereichs des Instrumentes an der äußeren Oberfläche des Beins des Patienten und damit ein Einschieben des Schafts der Spatelspitze dicht unter der Hautoberfläche entlang der Vene problemlos ermöglicht. Durch die erfindungsgemäß seitlich schräg angeordnete Okularmuschel ragt auch diese nicht mehr über die vom Handgriff abgewandte Außenseite des Instrumentes vor.

Durch die erfindungsgemäße Bauweise mit einer von Vorsprüngen freie Außenseite wird es möglich, das medizinische Instrument über die gesamte Länge seines Schafts in das Bein des Patienten einzuführen. Da Verdickungen, Wülste und ähnliches im proximalen Bereich des Instrumentes fehlen, kann das Instrument im Bereich des Einschnitts eng anliegend eingeführt und während der Operation so gehalten werden. Auf diese Weise erlaubt es das erfindungsgemäße Instrument, trotz eines kleinen Einschnitts die volle Länge des Schafts auszunutzen.

Diese einheitliche Fläche ermöglicht auch ein einfaches Einführen von weiteren Hilfsinstrumenten, beispielsweise VenenDissektoren, Präparier- bzw. Faßzangen, Scheren, Ligaturschlingen und ähnliches, ohne eine größere Inzision zu benötigen.

Die vorgenannte Außenseite des erfindungsgemäßen medizinischen Instruments muß nicht durchgehend einteilig sein. Sie kann aus mehreren hintereinander angeordneten Flächen gebildet sein, die zu verschiedenen Bauelementen des Instrumentes wie der Endoskopoptik, dem Handgriff und dem Schaft gehören, die gegebenenfalls von einander trennbar sind. Entscheidend ist dabei, daß die genannte Außenseite frei von Vorsprüngen ist, die den äußeren Umfang des Schafts deutlich überragen. Somit ist das medizinische Instrument an der am Bein des Patienten anliegenden Seite im wesentlichen flach ausgebildet, und das Instrument wird an dieser Außenseite entlang des Beins des Patienten in die Inzision geschoben.

Ein weiterer Vorteil des erfindungsgemäßen Instruments besteht darin, daß der Arzt unabhängig von der Einschubtiefe des Instruments sein Auge stets ungehindert an die Okularmuschel führen kann, da diese vom Schaft und dadurch vom Bein des Patienten absteht. Im Falle der Verwendung einer Kamera am Okular des Instrumentes wird das Einführen der Hilfsinstrumente vorteilhafterweise durch die angeschlossene Kamera nicht mehr behindert. Somit wird zusätzlich die Handhabung des erfindungsgemäßen Instrumentes auf vorteilhafte Weise verbessert.

Somit wird die der Erfindung zugrunde liegende Aufgabe vollkommen gelöst.

In einer bevorzugten Ausgestaltung weist der Handgriff einen Befestigungsabschnitt auf, der im oberen Bereich in Form einer Hülse ausgebildet ist, die den Schaft axial teilweise und auf der vom Handgriff abgewandten Außenseite des Schafts mit einer möglichst geringen Materialstärke umgreift.

Diese Maßnahme hat den Vorteil, einerseits eine stabile Verbindung zwischen dem Handgriff und dem Schaft zu bewerkstelligen, und andererseits die vom Handgriff abgewandte Außenseite des Instruments frei von Schultern, Stufen oder Vorsprüngen zu halten.

In einer weiteren bevorzugten Ausgestaltung bildet eine Längsmittelachse der Okularmuschel mit der Längsmittelachse des Schafts einen Winkel im Bereich von 30° bis 60°, vorzugsweise 45°.

Wenn die Okularmuschel in einem Winkel in diesem Bereich abstehend angeordnet ist, so kann der Arzt besonders bequem die Okularmuschel von der Seite des Instruments, die dem Körper des Patienten abgewandt ist, einsehen.

In einer weiteren bevorzugten Ausgestaltung ist die Okularmuschel an einem Okulargehäuse der Endoskopoptik angeordnet, daß eine der Okularmuschel abgewandte Außenseite aufweist, die mit der dem Handgriff abgewandten Außenseite des Schafts in etwa fluchtet.

Diese Maßnahme hat den Vorteil, daß die vorgenannte Außenseite des Okulargehäuses eine schulterfreie Verlängerung der vom Handgriff abgewandten Außenseite des Instruments bildet, wodurch die Führung des Instruments entlang des Beins durch die durch das Okulargehäuse gebildete verlängerte Anlagefläche verbessert ist.

In einer weiteren bevorzugten Ausgestaltung ist der Schaft als umfänglich geschlossener Hohlschaft zur Aufnahme eines sich bis zur Spatelspitze erstreckenden Optikschafts der Endoskopoptik ausgebildet.

Diese Maßnahme hat den Vorteil, daß der in dem Schaft aufgenommene Optikschaft der Endoskopoptik eine verbesserte Führung beim Einschieben entlang des Schaftes und einen verbesserten Halt in dem Schaft erfährt, so daß die Montage der Endoskopoptik an dem Schaft des Instruments erleichtert ist. Ein geschlossener Schaft mit einem innenliegenden Optikschaft hat darüber hinaus den Vorteil, daß die äußere Oberfläche des Schafts allseitig glatt und kantenfrei ausgebildet werden kann, wodurch sich der Schaft im Operationsgebiet leichter voranschieben läßt. Außerdem wird der Optikschaft vor Verschmutzungen geschützt. Weiterhin können zum Entfernen des Bindesgewebes und zum Abtrennen der Vene weitere Hilfsinstrumente in den Schaft des Instruments eingeführt werden. Alle diese Hilfsinstrumente werden dann von dem Schaft umschlossen und somit ebenfalls vor Verschmutzungen geschützt. Vor allem erfahren die Instrumente eine "ruckfreie" Führung am Schaft in Richtung distales Ende.

In einer weiteren bevorzugten Ausgestaltung ist die vom Handgriff abgewandte Außenseite des Schafts zur Längsmittelachse des Schafts hin gesehen im Querschnitt plan mit einer geringfügigen konkaven Wölbung ausgebildet.

Da die dem Handgriff abgewandte Außenseite des Schafts beim Einführen des Instruments entlang der äußeren Beinoberfläche geführt wird, hat diese Maßnahme den Vorteil, daß diese Außenseite flächig am Bein anliegt und somit eine verbesserte Führung des Schafts entlang des Beines ermöglicht. Die geringfügige konkave Ausgestaltung hat zusätzlich den Vorteil, daß der bereits in die Inzision eingeführte Abschnitts des Schaftes mit der Wölbung eine gewisse Zwangsführung entlang der Vene erfährt.

In einer weiteren bevorzugten Ausgestaltung ist eine dem Handgriff zugewandte Außenseite des Schafts zur Längsmittelachse des Schafts hin gesehen im Querschnitt konvex gewölbt.

Diese Maßnahme hat den Vorteil, daß der in dem Schaft aufgenommene Optikschaft beim Einschieben in den Schaft des Instruments in der Wölbung automatisch eine zentrierte Lage in dem Schaft einnimmt, so daß die Montage der Endoskopoptik an dem Schaft weiter vereinfacht wird.

In einer weiteren bevorzugten Ausgestaltung weist die Spatelspitze eine löffelförmige Wölbung auf, die sich zur dem Handgriff abgewandten Seite des Instruments hin öffnet.

Hierbei ist von Vorteil, daß im Bereich der distalen Spatelspitze beim Vorwärtsschieben des Instruments eine Operationshöhle gebildet wird, die durch die Endoskopoptik gut ausgeleuchtet und beobachtet werden kann. Die löffelförmige Wölbung der Spatelspitze schützt dabei den Bereich, in dem die distalen Elemente der Hilfsinstrumente, beispielsweise Maulteile von Zangen oder dergleichen betätigt werden.

In einer weiteren bevorzugten Ausgestaltung weist die Spatelspitze eine seitliche Verbreiterung auf, so daß sie den Schaft quer zu dessen Längsmittelachse zumindest einseitig überragt.

Diese Maßnahme hat den Vorteil, daß die von der Spatelspitze beim Voranschieben des Schaftes geschaffene Operationshöhle gegenüber der von der Spatelspitze des bekannten Instruments geschaffenen Operationshöhle vergrößert wird. Eine vergrößerte Operationshöhle hat den Vorteil, daß mehr Raum für die Maulteile der Hilfsinstrumente geschaffen wird.

In einer weiteren bevorzugten Ausgestaltung verjüngt sich die Spatelspitze zum distalen Ende hin.

Diese Verjüngung hat den Vorteil, daß sie das Voranschieben des erfindungsgemäßen Instruments durch das Körpergewebe hindurch erleichtert.

In einer weiteren bevorzugten Ausgestaltung steht der Handgriff von dem Schaft schräg zum distalen Ende hin ab.

Diese Maßnahme hat den Vorteil, daß das Instrument an dem somit in Vorschubrichtung geneigten Handgriff mit gerader Handhaltung und somit bequem und mit hoher Kraft in die Inzision eingeschoben werden kann, wodurch die Handhabung des erfindungsgemäßen Instruments weiter verbessert ist.

In einer weiteren bevorzugten Ausgestaltung schließen die gedachte erste Halbebene und die gedachte zweite Halbebene einen Winkel von weniger als 10°, vorzugsweise etwa 0°, ein.

Bei dieser Ausgestaltung stehen demnach der Handgriff und die Okularmuschel in einer gleichen Ebene von dem Schaft ab, wodurch der Vorteil erzielt wird, daß nach dem Einführen des Instruments dieses auch um seine Längsachse gedreht werden kann, ohne daß die Okularmuschel dabei ein Hindernis darstellt. Ein Drehen des Instruments beim Voranschieben kann bspw. dazu genutzt werden, um Seitenästen der Vena Saphena Magna beim Voranschieben des Instruments auszuweichen.

Weitere Vorteile ergeben sich aus der nachfolgenden Beschreibung und der beigefügten Zeichnung.

Es versteht sich, daß die vorstehend genannten und die nachstehend noch zu erläuternden Merkmale nicht nur in den angegebenen Kombinationen, sondern auch in anderen Kombinationen oder in Alleinstellung verwendbar sind, ohne den Rahmen der vorliegenden Erfindung zu verlassen.

Ein Ausführungsbeispiel der Erfindung wird nachstehend unter Bezugnahme auf die Zeichnung näher erläutert. Es zeigen:
- Fig. 1: eine Seitenansicht eines erfindungsgemäßen medizinischen Instrumentes, teilweise in einem Längsschnitt;
- Fig. 2: einen Schnitt durch das Instrument entlang der Linie II-II in Fig. 1 in vergrößertem Maßstab;
- Fig. 3: eine Draufsicht auf die von dem Instrument in Fig. 1 abgenommene Endoskopoptik;
- Fig. 4: eine schematische Darstellung zur Erläuterung des Verfahrens der Entnahme der Vena Saphena Magna aus einem Bein; und
- Fig. 5 und 6: das distale Ende eines Venen-Dissektors, der bei der Entnahme der Vena Saphena Magna verwendet wird, wobei Fig. 5 eine Draufsicht und Fig. 6 eine Vorderansicht ist.

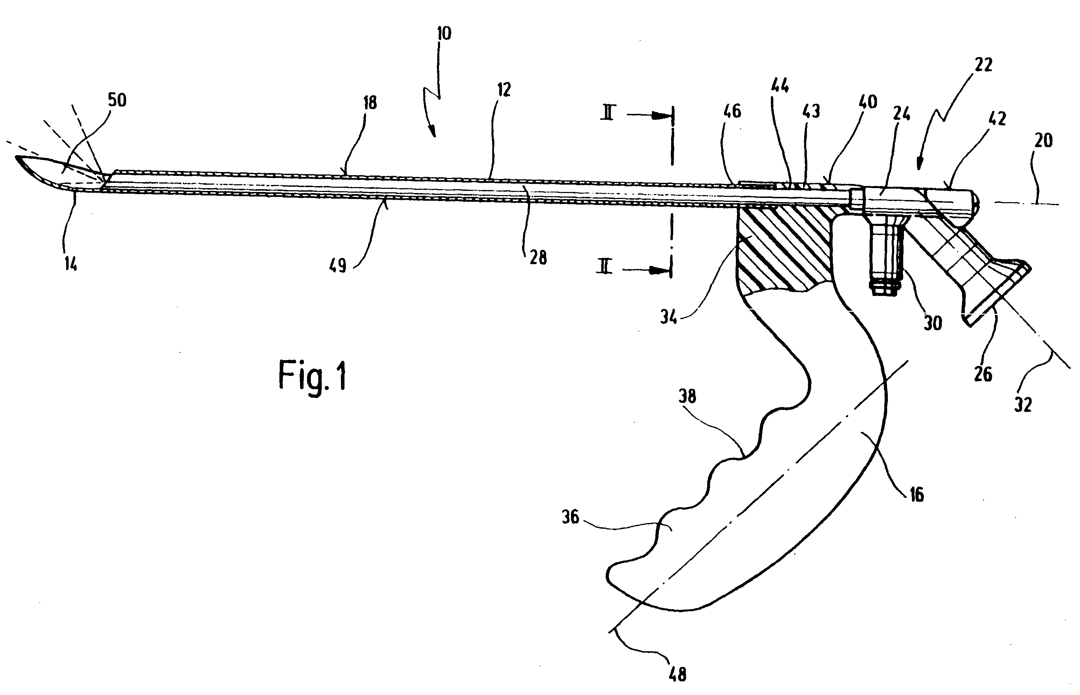

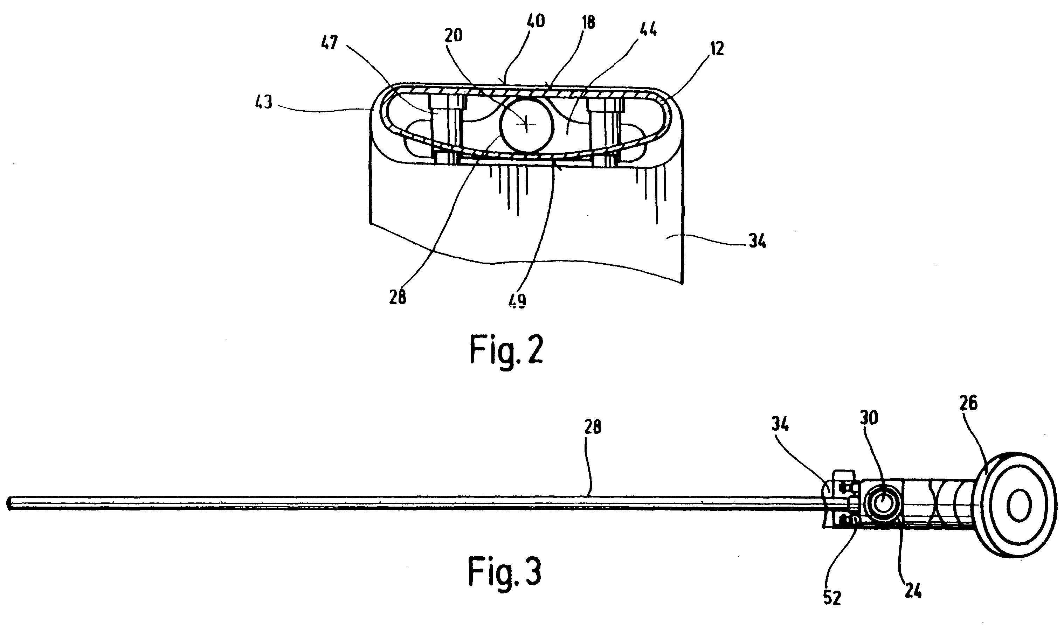

In Fig. 1 und 2 ist ein medizinisches Instrument zur Entnahme der Vena Saphena Magna dargestellt, das mit dem allgemeinen Bezugszeichen 10 versehen ist.

Das medizinische Instrument 10 weist einen langerstreckten Schaft 12 auf, der an seinem distalen Ende eine Spatelspitze 14 trägt, und in dessen proximalem Bereich ein Handgriff 16 angeordnet ist, der von dem Schaft 12 seitlich absteht.

Der Schaft 12 weist eine vom Handgriff 16 abgewandte Außenseite 18 auf. Die Außenseite 18 ist diejenige Seite, die beim Einführen des Schafts 12 in das Bein eines Patienten mit ihrem außerhalb der Inzision, d.h. außerhalb der Einstichstelle befindlichen Bereich an der Außenseite des Beins anliegt, und deren bereits eingeführter Bereich entlang der Vene geführt wird.

Wie aus Fig. 2 hervorgeht, ist die Außenseite 18 zu einer Längsmittelachse 20 des Schafts 10 hin gesehen im Querschnitt im wesentlichen plan mit einer geringfügigen konkaven Wölbung ausgebildet.

Das Instrument 10 weist ferner eine von dem Schaft 12 und dem Handgriff 16 abnehmbare Endoskopoptik 22 auf, die in Fig. 3 in Alleinstellung im vom Schaft 12 abgenommenen Zustand dargestellt ist.

Die Endoskopoptik 22 weist am proximalen Ende ein Okulargehäuse 24 mit einer Okularmuschel 26 auf. An das Okulargehäuse 24 schließt sich distalseitig ein Optikschaft 28 an. Der Optikschaft 28 ist als zylindrisches Rohr ausgebildet, in dem ein optisch abbildendes System angeordnet ist, das aus einem Linsensystem, Blenden, Filtern etc. oder aus einem geordneten Lichtleitfaserbündel besteht. Weiterhin ist in dem Optikschaft 28 ein lichtzuführendes Faserbündel angeordnet, mit dem Licht in das Operationsgebiet zugeführt wird. Dazu ist an dem Okulargehäuse 24 ein Anschluß 30 zum Anschließen eines nicht dargestellten, mit einer nicht dargestellten Lichtquelle verbindbaren Lichtleitkabels vorhanden.

Die Okularmuschel 26 ist im an dem Schaft 12 montierten Zustand der Endoskopoptik 22 zur gleichen Seite wie der Handgriff 16 bezüglich der Längsmittelachse 20 des Schafts 12 zum proximalen Ende hin schräg gerichtet angeordnet. Der Handgriff 16 schließt mit der Okularmuschel 26 bezüglich der Längsmittelachse 20 einen Winkel von weniger als 90°, im gezeigten Ausführungsbeispiel von 0° ein. Mit anderen Worten ist die Okularmuschel 26 bezüglich des Handgriffs 16 so angeordnet, daß eine erste gedachte Halbebene, die sich von der Längsmittelachse 20 des Schafts 12 erstreckt und eine Längsmittelachse 32 der Okularmuschel 26 einschließt, mit einer gedachten zweiten Halbebene, die sich ebenfalls von der Längsmittelachse 20 des Schafts 12 erstreckt und eine Längsmittelachse 48 des Handgriffs 16 einschließt, einen Winkel von weniger als 90° einschließt.

Dabei bildet die Längsmittelachse 32 der Okularmuschel 26 mit der Längsmittelachse 20 des Schaftes 12 einen Winkel im Bereich von 30° bis 60°, in Fig. 1 einen Winkel von etwa 45°.

Der Anschluß 30 zum Anschließen des Lichtleitkabels steht etwa rechtwinklig zur gleichen Seite wie der Handgriff 16 bzw. wie die Okularmuschel 26 von dem Instrument 10 ab.

Der Handgriff 16 besteht aus einem Befestigungsabschnitt 34, der etwa rechtwinklig zu dem Schaft 12 verläuft, und aus einem eigentlichen Griffabschnitt 36, der Fingermulden 38 aufweist.

Der Handgriff 16 ist mit dem Schaft 12 so verbunden, daß die von dem Handgriff 16 abgewandte Außenseite 18 des Schafts 12 im Bereich des Befestigungsabschnitts 34 des Handgriffs 16 mit einer Außenseite 40 des Befestigungsabschnitts 34 eine im wesentlichen gleichmäßige Fläche bildet, die im wesentlichen frei von Vorsprüngen oder Schultern ist.

Ebenso weist das Okulargehäuse 24 eine entsprechende Außenseite 42 auf, die sich an die Außenseite 40 des Befestigungsabschnitts 34 des Handgriffs 16 proximal anschließt und somit mit der Außenseite 18 des Schafts in etwa fluchtet.

Die gesamte sich aus den Außenseiten 18, 40, 42 zusammensetzende Außenseite des Instruments 10 weist somit vom distalen bis zum proximalen Ende eine gleichmäßige Fläche auf, d.h. eine Fläche, die keine Unregelmäßigkeiten in Form von Schultern oder Vorsprüngen aufweist.

Der Befestigungsabschnitt 34 weist im oberen Bereich eine Ausgestaltung in Form einer Hülse 43 mit einer axial durchgehenden Öffnung 44 auf, durch die der Optikschaft 28 durchgeführt ist. Im distalen Bereich des Befestigungsabschnitts 34 ist ein Abschnitt 46 der Öffnung 44 entsprechend der Außenkontur des Schafts 12 ausgebildet, so daß der Schaft 12 in dem Befestigungsabschnitt 34 des Handgriffs 16 distalseitig einsteckbar ist.

Mittels durch den Befestigungsabschnitt 34 und den Schaft 12 durchgehender Schrauben 47, die zur Außenseite 40 hin nicht überstehen, sind der Schaft 12 und der Handgriff 16 unverlierbar miteinander verbunden. Auf der vom Handgriff 16 abgewandten Außenseite 18 umgreift die Hülse 43 des Befestigungsabschnitts 34 den Schaft 12 mit einer geringen Materialstärke, so daß der Befestigungsabschnitt 34 den Schaft 12 auf der Außenseite 18 im wesentlichen nicht überragt. Die zuvor genannte Materialstärke weist gerade noch ein für die sichere Befestigung des Handgriffs 16 an dem Schaft 12 erforderliches Maß auf.

Der Handgriff 16, genauer gesagt der Griffabschnitt 36 des Handgriffs 16 steht von dem Schaft 12 schräg zum distalen Ende hin ab, so daß die Längsmittelachse 48 des Handgriffs 16 mit der Längsmittelachse 20 des Instruments 10 zum distalen Ende hin gesehen einen Winkel von etwa 45° bildet.

Wie aus Fig. 2 weiter hervorgeht, ist der Schaft 12 als umfänglich geschlossener Hohlschaft ausgebildet, in dessen Inneren der Optikschaft 28 der Endoskopoptik 22 aufgenommen ist.

Eine dem Handgriff 16 zugewandte Außenseite 49 des Schafts 12, die der Außenseite 18 gegenüberliegt, ist zur Längsmittelachse 20 hin gesehen im Querschnitt konvex gewölbt.

Durch diese konvexe Wölbung der Außenseite 49 und auch durch die geringfügige konkave Wölbung der Außenseite 18 ist der Optikschaft 28 in dem Schaft 12 bezüglich der Längsmittelachse 20 zentriert aufgenommen.

Insgesamt ist der Schaft 12 im Querschnitt flach oval oder ganz leicht nierenförmig ausgebildet.

Beidseits des Optikschafts 28 ist noch ein axial durchgehender offener Raum in dem Schaft 12 zum Einführen von Hilfsinstrumenten vorhanden, die zur Entfernung der Vena Saphena Magna verwendet werden, wie Venen-Dissektoren, Faßzangen oder dergleichen.

Der Endoskopschaft 28 reicht distal bis zu der Spatelspitze 14. Die Spatelspitze 14 weist eine löffelförmige Wölbung auf, die sich zur Außenseite 18 des Schafts 12 hin öffnet. Weiterhin verjüngt sich die Spatelspitze 14 zum distalen Ende hin. Eine seitliche Verbreiterung 50 ist derart ausgebildet, daß die Spatelspitze 14 den Schaft 12 zur Außenseite 18 hin geringfügig überragt.

Zur schnell lösbaren Befestigung und Verriegelung der Endoskopoptik 22 an dem Befestigungsabschnitt 34 des Handgriffs 16 sind an dem Okulargehäuse 24 zwei axial vorstehende Stifte 52 vorgesehen, die mit entsprechenden Ausnehmungen in dem Befestigungsabschnitt 34 des Handgriffs 16 in Eingriff gebracht und verriegelt werden können.

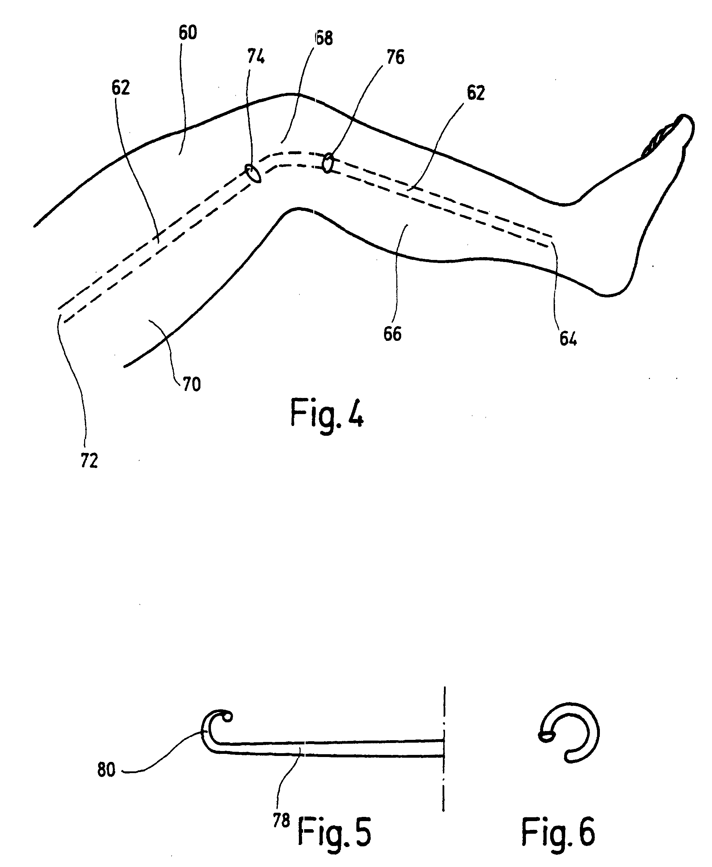

Anhand von Fig. 4 wird nun ein Verfahren zur Entnahme der Vena Saphena Magna beschrieben, bei dem das Instrument 10 verwendet wird.

In Fig. 4 ist das linke Bein 60 eines Patienten schematisch dargestellt. Die Vena Saphena Magna 62, die in Fig. 4 mit unterbrochenen Linien angedeutet ist, erstreckt sich subkutan vom Knöchelbereich 64 durch den Unterschenkel 66, am Knie 68 vorbei und durch den Oberschenkel 70 bis in die Leistengegend 72. Die Vena Saphena Magna 62 verläuft dabei auf der Innenschenkelseite des Beins 60.

Das hiernach beschriebene Entnahmeverfahren ermöglicht die Entnahme der Vena Saphena Magna 62 durch zwei Inzisionen 74 und 76, prinzipiell sogar durch nur eine der Inzisionen 74 oder 76.

Nach der Narkose wird der Patient auf dem Operationstisch auf dem Rücken liegend positioniert, wobei das Bein 70 leicht nach außen rotiert wird.

Soll die Vena Saphena Magna 62 hauptsächlich aus dem Oberschenkel 70 und nur teilweise aus dem Unterschenkel 66 entnommen werden, wird nur die Inzision 74 benötigt, die mittels eines Skalpells geringfügig oberhalb des Knies 68 als Querinzision eingebracht wird. Soll die Vena Saphena Magna 62 hauptsächlich aus dem Unterschenkel 62 und nur teilweise aus dem Oberschenkel 70 entnommen werden, wird nur die Inzision 76 benötigt, die geringfügig unterhalb des Knies 68 als Querinzision eingebracht wird.

Soll die gesamte Vena Saphena Magna 62 vom Knöchelbereich 64 bis zur Leistengegend 72 entnommen werden, ist es günstiger, wenn beide Inzisionen 74 und 76 eingebracht werden.

Unter Querinzision ist dabei zu verstehen, daß die Schnitte quer zur Längsrichtung des Oberschenkels 70 bzw. zur Längsrichtung des Unterschenkels 66 vorgenommen werden. Die Länge der Schnitte beträgt dabei etwa 2 bis 3 cm.

Die Inzisionen 74 bzw. 76 befinden sich, wie aus Fig. 4 hervorgeht, unmittelbar im Bereich der Vena Saphena Magna 62.

Die Inzision 74 und/oder 76 wird zunächst bis zur Vena Saphena Magna 62 hin frei präpariert.

Das Instrument 10 in Fig. 1 bis 3 wird nun mit der Endoskopoptik 22 bestückt. An die Okularmuschel 26 wird über einen Adapter eine Videokamera angeschlossen, die mit einem Monitor verbunden ist, auf dem das endoskopische Bild beobachtet wird.

Nun wird mit der Mobilisierung der Vena Saphena Magna 62 im Oberschenkel begonnen, wobei im ersten Schritt mittels des Instruments 10 in Fig. 1 bis 3 ein subkutaner Kanal bzw. Hohlraum entlang der Vena Saphena Magna geschaffen wird.

Das Instrument 10 wird dazu mit der Spatelspitze 14 voran in die Inzision 74 eingesetzt. Dabei liegt die dem Handgriff 16 abgewandte Außenseite 18 des Schafts 12 am Knie 68 an, und die Spatelspitze 14 zeigt in Richtung der Leistengegend 72.

Das Instrument 10 wird nun unter endoskopischer Sichtkontrolle auf dem Monitor langsam vorsichtig entlang der Vena Saphena Magna 62 in Richtung der Leistengegend 72 vorangeschoben.

Die Spatelspitze 14 schafft dabei entlang der Vena Saphena Magna 62 einen subkutanen Kanal bzw. Hohlraum. Beim Voranschieben der Spatelspitze 14 ist durch die endoskopische Sichtkontrolle sicherzustellen, daß keine ungewünschten subkutanen Nebenkanäle geschaffen werden.

Um beim Voranschieben des Instruments 10 Seitenästen der Vena Saphena Magna 62 auszuweichen, wird das Instrument 10 beim Voranschieben entlang der Vena Saphena Magna 62 entsprechend geringfügig gedreht.

Das Instrument 10 wird, wenn die Vena Saphena Magna 62 bis zur Leistengegend 72 entnommen werden soll, solange entlang der Vene 62 vorangeschoben, bis die Spatelspitze 14 die Leistengegend 72 erreicht hat, andernfalls wird an der beabsichtigten Endstelle der Entnahme haltgemacht.

Nun ist entlang der Vena Saphena Magna 62 ein subkutaner Kanal geschaffen worden, und im folgenden wird die Vena Saphena Magna 62 von ihren Seitenästen getrennt.

Dazu werden bei weiterhin eingesetztem Instrument 10 nun zusätzliche Instrumente, wie Scheren, in die Inzision 74 eingeführt, um die Vena Saphena Magna 62 von ihren Seitenästen fern von der Vena Saphena Magna 62 freizuschneiden.

Vor dem Durchschneiden der Seitenäste werden diese mittels Klemmen, die über einen Klemmenapplikator (nicht dargestellt) an Ort und Stelle gebracht werden, abgeklemmt, um den Blutfluß zu unterbrechen.

Zum Schneiden eignen sich insbesondere hochfrequenzstromgestützte Instrumente, wie Bipolar- oder Monopolarscheren, da bei Verwendung solcher Instrumente das Auftreten von Blutungen weitestgehend vermieden werden kann. Die Seitenaststümpfe können unter der Wirkung des Hochfrequenzstroms nämlich gleichzeitig koaguliert werden.

Nachdem die Vena Saphena Magna 62 im Oberschenkel 70 von ihren Seitenästen getrennt wurde, wird bei weiterhin eingesetztem Instrument 10 ein in Fig. 5 und 6 dargestellter Venendissektor 78 eingeführt, dessen distales Ende eine quer zur Längsrichtung des Instruments etwa halb- oder dreiviertelkreisförmig umgebogene Öse 80 aufweist.

Die Öse 80 wird nach Einsetzen durch die Inzision 74 um die Vena Saphena Magna 62 gelegt, und das Instrument 78 wird dann entlang der Vena Saphena Magna 62 bis zur Leistengegend 72 vorangeschoben, wodurch noch anhaftendes subkutanes Gewebe von der Vena Saphena Magna 62 abgestreift wird.

Die Vena Saphena Magna 62 ist nun vollkommen mobilisiert, aber an ihrem Ende in der Leistengegend 92 noch nicht durchtrennt.

Alle vorgenannten Vorgänge, nämlich das Trennen der Vena Saphena Magna 62 von ihren Seitenästen und das Trennen der Vena Saphena Magna 62 von dem anliegenden subkutanen Gewebe erfolgt unter ständiger Sichtkontrolle auf dem Monitor durch die Endoskopoptik 22 des Instruments 10, das bei diesen Vorgängen im Operationsgebiet eingesetzt bleibt. Das Instrument 10 wird dabei durch Voranschieben oder Zurückziehen jeweils so positioniert, daß die Spatelspitze 14 sich jeweils an derjenigen Stelle befindet, an der gerade präpariert wird.

Die löffelartig verbreiterte Ausgestaltung der Spatelspitze 14, insbesondere die Verbreiterung 50 bildet dabei jeweils einen Hohlraum, in dem mit dem Applikator, jeweiligen Schneidinstrument oder Dissektor wie vorstehend beschrieben dann entsprechend sicher gearbeitet werden kann.

Nach der vollständigen Mobilisierung der Vena Saphena Magna 62 im Oberschenkel wird das Instrument 10 aus der Inzision 74 genommen und wieder die Inzision 74 eingeführt, jedoch mit der Spatelspitze 14 in Richtung Knöchelbereich 64 zeigend, wonach die gleichen vorbeschriebenen Vorgänge zur Mobilisierung der Vena Saphena Magna 62 im Unterschenkel durchgeführt werden.

Soll bis zum Knöchelbereich 64 entnommen werden, eignet sich hierfür die Inzision 76 besser.

Nach der vollständigen Mobilisierung der Vena Saphena Magna 62 im Unterschenkel 66 wird die Vena Saphena Magna 62 durch die Inzision 76 bzw. 74 geringfügig vorgezogen. Um den vorgezogenen Abschnitt wird ein Faden gelegt, der zu einer zuziehbaren Schlinge geknotet wird.

Die noch nicht zugezogene Schlinge wird mittels des Venendissektors 78 unter endoskopischer Kontrolle durch das Instrument 10 dann entlang der Vena Saphena Magna 62 bis zum Knöchelbereich 64 geschoben.

Am Knöchel 74 wird die Schlinge dann zugezogen, um die Vena Saphena Magna 62 am Knöchelbereich 64 abzubinden.

Vom Knie 68 aus gesehen vor der Schlinge wird dann die Vena Saphena Magna 62 mit einer Schere durchtrennt. Der abgetrennte Unterschenkelabschnitt der Vena Saphena Magna 62 kann dann aus der Inzision 74 oder 76 herausgezogen werden.

Der gleiche vorgenannte Schritt wird dann im Oberschenkel 70 durchgeführt, um die Vena Saphena Magna 62 im Bereich der Leistengegend 72 abzubinden und an einer Stelle davor abzutrennen. Nun ist die Vena Saphena Magna 62 vollkommen abgetrennt und wird aus der Inzision 76 bzw. 74 vollständig aus dem Bein 60 herausgezogen.

Die so entnommene Vena Saphena Magna 62 steht dann für eine Bypass-Operation zur Verfügung.

Bis zur Verwendung in der Bypass-Operation kann die Vena Saphena Magna 62 entsprechend in einer Lösung aufbewahrt werden.

Die Inzision 74 bzw. die Inzision 76 wird entsprechend anschließend genäht und das Bein 60 für 24 Stunden mit einer elastischen Bandage gewickelt.

## Patentansprüche

1. Medizinisches Instrument zur endoskopischen Entnahme der Vena Saphena Magna, mit einem langerstreckten Schaft (12), der am distalen Ende eine Spatelspitze (14) aufweist, und in dessen proximalem Bereich ein seitlich abstehender Handgriff (16) angeordnet ist, und ferner mit einer Endoskopoptik (22), die eine Okularmuschel (26) aufweist, die am proximalen Ende des Instruments (10) angeordnet ist, wobei sich von einer Längsmittelachse (20) des Schafts (12) eine gedachte erste Halbebene erstreckt, die eine Längsmittelachse (32) der Okularmuschel (26) einschließt, **dadurch gekennzeichnet**, daß der Handgriff (16) so mit dem Schaft (12) verbunden ist, daß eine von dem Handgriff (16) abgewandte Außenseite (18, 40) des Instruments (10) vom distalen Ende bis zum proximalen Ende durchgehend eine von Vorsprüngen im wesentlichen freie gerade Fläche aufweist, und daß die Okularmuschel (26) bezüglich der Längsmittelachse (20) des Schafts (12) schräg gerichtet angeordnet ist, wobei sich von der Längsmittelachse (20) des Schafts (12) eine gedachte zweite Halbebene erstreckt, die eine Längsmittelachse (48) des Handgriffs (16) einschließt, und daß die gedachte erste Halbebene und die gedachte zweite Halbebene einen Winkel von weniger als 90° einschließen.

2. Instrument nach Anspruch 1, **dadurch gekennzeichnet**, daß der Handgriff (16) einen Befestigungsabschnitt (34) aufweist, der im oberen Bereich in Form einer Hülse ausgebildet ist, die den Schaft (12) axial teilweise und auf der vom Handgriff (16) abgewandten Außenseite (18) des Schafts (12) mit einer möglichst geringen Materialstärke umgreift.

3. Instrument nach Anspruch 1 oder 2, **dadurch gekennzeichnet**, daß eine Längsmittelachse (32) der Okularmuschel (26) mit der Längsmittelachse (20) des Schafts (12) einen Winkel im Bereich von 30° bis 60", vorzugsweise 45°, bildet.

4. Instrument nach einem der Ansprüche 1 bis 3, **dadurch gekennzeichnet**, daß die Okularmuschel (26) an einem Okulargehäuse (24) der Endoskopoptik (22) angeordnet ist, das eine der Okularmuschel (26) abgewandte Außenseite (42) aufweist, die mit der dem Handgriff (16) abgewandten Außenseite (18) des Schafts (12) in etwa fluchtet.

5. Instrument nach einem der Ansprüche 1 bis 4, **dadurch gekennzeichnet**, daß der Schaft (12) als umfänglich geschlossener Hohlschaft zur Aufnahme eines sich bis zur Spatelspitze (14) erstreckenden Optikschafts (28) der Endoskopoptik (22) ausgebildet ist.

6. Instrument nach einem der Ansprüche 1 bis 5, **dadurch gekennzeichnet**, daß die dem Handgriff (16) abgewandte Außenseite (18) des Schafts (12) zur Längsmittelachse (20) des Schafts (12) hin gesehen im Querschnitt mit einer geringfügigen konkaven Wölbung ausgebildet ist.

7. Instrument nach einem der Ansprüche 1 bis 6, **dadurch gekennzeichnet**, daß eine dem Handgriff (16) zugewandte Außenseite (49) des Schafts (12) zur Längsmittelachse (20) des Schafts (12) hin gesehen im Querschnitt konvex gewölbt ist.

8. Instrument nach einem der Ansprüche 1 bis 7, **dadurch gekennzeichnet**, daß die Spatelspitze (14) eine löffelförmige Wölbung aufweist, die sich zur dem Handgriff (16) abgewandten Außenseite (18, 40) des Instruments (10) hin öffnet.

9. Instrument nach einem der Ansprüche 1 bis 8, **dadurch gekennzeichnet**, daß die Spatelspitze (14) eine seitliche Verbreiterung (50) aufweist, so daß sie den Schaft (12) quer zu dessen Längsmittelachse (20) zumindest einseitig überragt.

10. Instrument nach einem der Ansprüche 1 bis 9, **dadurch gekennzeichnet**, daß sich die Spatelspitze (14) zum distalen Ende hin verjüngt.

11. Instrument nach einem der Ansprüche 1 bis 10, **dadurch gekennzeichnet**, daß der Handgriff (16) von dem Schaft (12) schräg zum distalen Ende hin absteht.

12. Instrument nach einem der Ansprüche 1 bis 11, **dadurch gekennzeichnet**, daß die gedachte erste Halbebene und die gedachte zweite Halbebene einen Winkel von weniger als 10°, vorzugsweise etwa 0°, einschließen.

## Claims

1. A medical instrument for endoscopic removal of the great saphenous vein, comprising an elongated shaft (12) which has at the distal end a spatula tip (14) and in whose proximal region is arranged a laterally projecting handle (16), further comprising an endoscopic optical system (22) which comprises an eyepiece cup (26) that is arranged at the proximal end of the instrument (10), wherein a first imaginary half plane extends from a longitudinal center axis (20) of the shaft (12), **characterized in that** the handle (16) is joined to the shaft (12) in such a way that an outer side (18, 40) of the instrument (10) facing away from the handle (16) continuously has from the distal to the proximal end a substantially straight surface which is free of projections, and that the eyepiece cup (26) is arranged in oblique orientation with respect to the longitudinal center axis (20) of the shaft (12), wherein a second imaginary half plane which encloses a longitudinal center axis (48) of the handle (16) extends from the longitudinal center axis (20) of the shaft (12), and that the first imaginary half plane and the second imaginary half plane enclose an angle of less than 90°.

2. The instrument of claim 1, **characterized in that** the handle (16) has an attachment segment (34) that is configured in the upper region in the form of a sleeve which axially partially fits over the shaft (12) with the least possible material thickness on the outer side (18) of the shaft (12) facing away from the handle (16).

3. The instrument of claim 1 or 2, **characterized in that** a longitudinal center axis (32) of the eyepiece cup (26) forms an angle in the range from 30° to 60°, preferably 45°, with the longitudinal center axis (20) of the shaft (12).

4. The instrument of one of claims 1 through 3, **characterized in that** the eyepiece cup (26) is arranged on an eyepiece housing (24) of the endoscopic optical system (22) which has an outer side (42) facing away from the eyepiece cup (26), that approximately aligns with the outer side (18) of the shaft (12) facing away from the handle (16).

5. The instrument of one of claims 1 through 4, **characterized in that** the shaft (12) is configured as a circumferentially closed hollow shaft for reception of an optical shaft (28) extending to the spatula tip (14), of the endoscopic optical system (22).

6. The instrument of one of claims 1 through 5, **characterized in that** the outer side (18) of the shaft (12) facing away from the handle (16) is configured with a slight concave curvature in cross section when viewed toward the longitudinal center axis (20) of the shaft (12).

7. The instrument of one of claims 1 through 6, **characterized in that** an outer side (49) of the shaft (12) facing toward the handle (16) is convexly curved in cross section when viewed toward the longitudinal center axis (20) of the shaft (12).

8. The instrument of one of claims 1 through 7, **characterized in that** the spatula tip (14) comprises a spoon-shaped curvature that opens toward the outer side (18, 40) of the instrument (10) facing away from the handle (16).

9. The instrument of one of claims 1 through 8, **characterized in that** the spatula tip (14) has a lateral widening (50) so that it extends beyond the shaft (12), at least on one side, transversely to its longitudinal center axis (20).

10. The instrument of one of claims 1 through 9, **characterized in that** the spatula tip (14) tapers toward the distal end.

11. The instrument of one of claims 1 through 10, **characterized in that** the handle (16) projects from the shaft (12) obliquely toward the distal end.

12. The instrument of one of claims 1 through 11, **characterized in that** the imaginary first half plane and the imaginary second half plane enclose an angle of less than 10°, preferably approximately 0°.

## Revendications

1. Instrument médical pour l'ablation de la grande veine saphène, comportant une tige (12) allongée, qui présente à son extrémité distale une pointe en spatule (14) et dans la zone proximale de laquelle est disposée une poignée (16) dépassant latéralement, et comportant en outre une optique d'endoscope (22) qui comporte une bonnette d'oculaire (26), laquelle est disposée à l'extrémité proximale de l'instrument (10), un premier demi-plan imaginaire, qui contient un axe médian longitudinal (32) de la bonnette d'oculaire (26), s'étendant depuis un axe médian longitudinal (20) de la tige (12), **caractérisé en ce que** la poignée (16) est reliée à la tige (12) de manière qu'un côté extérieur (18, 40) de l'instrument (10), tourné à l'opposé de la poignée (16), présente une surface rectiligne, sensiblement exempte de saillies d'un bout à l'autre, depuis l'extrémité distale jusqu'à l'extrémité proximale, et en ce que la bonnette d'oculaire (26) est disposée orientée obliquement par rapport à l'axe médian longitudinal (20) de la tige (12), un deuxième demi-plan imaginaire, qui contient un axe médian longitudinal (48) de la poignée (16), s'étendant depuis l'axe médian longitudinal (20) de la tige (12), et en ce que le premier demi-plan imaginaire et le deuxième demi-plan imaginaire forment un angle de moins de 90°.

2. Instrument selon la revendication 1, **caractérisé en ce que** la poignée (16) présente une portion de fixation (34) qui, dans la zone supérieure, a la forme d'une douille, laquelle entoure partiellement la tige (12) axialement, et sur le côté extérieur (18) de la tige (12) tourné à l'opposé de la poignée (16), avec une épaisseur de matière aussi faible que possible.

3. Instrument selon la revendication 1 ou 2, **caractérisé en ce qu**'un axe médian longitudinal (32) de la bonnette d'oculaire (26) forme, avec l'axe médian longitudinal (20) de la tige (12), un angle compris entre 30° et 60°, de préférence de 45°.

4. Instrument selon l'une des revendications 1 à 3, **caractérisé en ce que** la bonnette d'oculaire (26) est disposée sur un boîtier d'oculaire (24) de l'optique d'endoscope (22), lequel présente un côté extérieur (42) qui est tourné à l'opposé de la bonnette d'oculaire (26) et qui est approximativement aligné avec le côté extérieur (18) de la tige (12) tourné à l'opposé de la poignée (16).

5. Instrument selon l'une des revendications 1 à 4, **caractérisé en ce que** la tige (12) est conformée en tige creuse fermée périphériquement, destinée à recevoir une tige d'optique (28) de l'optique d'endoscope (22), s'étendant jusqu'à la pointe en spatule (14).

6. Instrument selon l'une des revendications 1 à 5, **caractérisé en ce que** le côté extérieur (18) de la tige (12), tourné à l'opposé de la poignée (16), vu vers l'axe médian longitudinal (20) de la tige (12), présente une section avec une courbure légèrement concave.

7. Instrument selon l'une des revendications 1 à 6, **caractérisé en ce qu**'un côté extérieur (49) de la tige (12), tourné vers la poignée (16), vu vers l'axe médian longitudinal (20) de la tige (12), présente une section convexe.

8. Instrument selon l'une des revendications 1 à 7, **caractérisé en ce que** la pointe en spatule (14) présente une courbure en forme de cuiller qui est ouverte vers le côté extérieur (18, 40), opposé à la poignée (16), de l'instrument (10).

9. Instrument selon l'une des revendications 1 à 8, **caractérisé en ce que** la pointe en spatule (14) présente un élargissement latéral (50), ce qui fait qu'elle dépasse de la tige (12), transversalement à son axe médian longitudinal (20), au moins d'un côté.

10. Instrument selon l'une des revendications 1 à 9, **caractérisé en ce que** la pointe en spatule (14) se rétrécit vers l'extrémité distale.

11. Instrument selon l'une des revendications 1 à 10, **caractérisé en ce que** la poignée (16) dépasse de la tige (12), obliquement par rapport à l'extrémité distale.

12. Instrument selon l'une des revendications 1 à 11, **caractérisé en ce que** le premier demi-plan imaginaire et le deuxième demi-plan imaginaire forment un angle inférieur à 10°, de préférence d'environ 0°.
